# EUROPEAN PATENT SPECIFICATION

(11) **EP 0 604 399 B1**
(45) Date of publication and mention of the grant of the patent: **02.12.1998**
(21) Application number: 94102959.7
(22) Date of filing: 23.05.1991
(51) Int. Cl.: A61M 16/10, A61M 16/08, G01N 1/22, A61B 5/097

(54) **Y-piece connector for ventilator**
Y-förmiges Verbindungsstück für Beatmungsgerät
Connecteur en forme de pièce Y pour ventilateur artificiel

(30) Priority: 18.06.1990 SE 9002162; 02.11.1990 SE 9003505
(43) Date of publication of application: 29.06.1994
(62) Divisional of application: 91108316.0
(73) Proprietor: ENGSTRÖM MEDICAL AB, S-161 02 Bromma (SE)
(72) Inventor: Kihlberg, Ake, S-183 50 Täby (SE); Tryggvason, Ragnar, S-240 21 Löddeköpinge (SE); Wikefeldt, Per, S-175 45 Järfälla (SE)
(74) Representative: Lettström, Richard Wilhelm

(56) References cited:
- EP-A- 0 265 163
- WO-A-89/04684
- WO-A-90/04425
- FR-A- 1 121 482
- FR-A- 2 315 955
- GB-A- 2 116 434
- US-A- 4 456 014
- US-A- 4 558 708

## Description

### TECHNICAL FIELD

The present invention relates to a connection arrangement for connecting a patient to a respirator, anaesthesia machine or similar, of the type mentioned in the preamble of claim 12 and a Y-piece for the type mentioned in the preamble of claim 1 for use in such a connection arrangement.

From the following description it will be evident that the expression "Y-piece" also includes shapes other than a conventional "Y". Of importance is only that said patient attachment piece is able to be connected to an inhalation resp an exhalation tube. The expression "Y-piece" also includes for example T-connections and even connections for connecting coaxially arranged inhalation and exhalation tubes.

### BACKGROUND

A Y-piece with a sample withdrawal outlet extending perpendicular to the flow of the air in the Y-piece is known from US-A 4 558 708 which is used as a basis for the preambles of claims 1 and 12, respectively.

The patient attachment piece can by way of example be designed in accordance with US-A-4 516 573. In this patent there is described a bellowed patient attachment piece which contains a wad of moisture and heat absorbing material. In this way heat and moisture is taken up from the exhaled gas and given to the inhaled gas. Such patient attachment pieces are often connected with various types of Y-pieces and are possibly also equipped with various types of bacteria filters. The more components which are connected leads to a bulkier construction which can be inconvenient for the patient. Furthermore, the connection of a plurality of components leads to the risk of, on the one hand, leakage and, on the other hand, incorrectly connected components.

Another problem is that the so called dead volume for the breathing gas increases when a plurality of components are combined. The same is also often true for samples taken at a distance from the patient. WO-A-8904864 describes a sample withdrawal tube which samples exhaled air after it has passed through a bacteria filter in a regenerative heat and moisture exchanger. However, the withdrawal tube samples air which can contain unfiltered inhalation air from the dead volume of the exchanger as well as filtered exhalation air from the patient.

With the attachment of a patient to a respirator, anaesthesia machine or similar, the need arises for a continuous or intermittent sampling of the exhaled gas respectively a proximal pressure measuring. This should take place as close to the patient as possible and can occur for example in the way described in US-A-4 838 258, i.e. with the help of a tube which extends from the respirator itself through the exhalation tube to a point near the patient. The disadvantage with the construction described in this patent is, however, that the withdrawn sample can be, on the one hand, very moist, and, on the other hand, contaminated with bacteria.

### DESCRIPTION OF THE INVENTION

The above mentioned problems are reduced or eliminated according to the present invention which relates to a Y-piece, for use in a connection arrangement for connecting a patient to a respirator, anaesthesia machine or similar, wherein said Y-piece comprises a sample withdrawal outlet wherein a bacteria filter is mounted in said Y-piece substantially perpendicular to the through-flow direction, and the sample withdrawal outlet is arranged in the exhalation flow direction after the bacteria filter and is connected to a sample withdrawal tube which extends from the bacteria filter to the outlet and is arranged so as to take bacteria-free samples through said filter. The present invention also relates to connection arrangement for connecting a patient to a respirator, anaesthesia machine or similar, comprising a patient attachment piece, a Y-piece, arranged to join the patient attachment piece to an inhalation resp exhalation tube, wherein the Y-piece is a Y-piece of the type described above. In this way considerably dryer gas samples are obtained without secretion from the patient. The proximal pressure of the patient can also be measured via this outlet.

In order to reduce the dead volume the bacteria filter is arranged within the Y-piece itself.

In order that samples can be taken from as close to the patient as possible, the first sample withdrawal tube can via the bacteria filter be connected to a second sample withdrawal tube which extends from the filter towards the patient attachment piece and preferably up to and, possibly into this piece. The filtering of the withdrawn samples is hereby simplified, if the said first and second sample withdrawal tubes are arranged on either side of the bacteria filter and are in pressurized contact therewith via a at right angles to the flow direction widened portion, for example a cone-shaped and/or cylindrical funnel-like portion.

The moisture content of the withdrawn sample is reduced, if the patient attachment piece contains a heat and moisture exchange system in the form of a wad or similar of a flexible material, such as fibres, with the ability to take heat and moisture from exhaled gas and subsequently deliver this to the inhaled gas. Preferably the complete patient attachment piece is made from a flexible material. Such a heat and moisture exchange system is disclosed in US-A-4 516 573.

In order to reduce the breathing resistance, the through-flow area of the Y-piece can hereby be enlarged in comparison with that of the patient attachment piece. By reducing the number of parts included in the connection arrangement, choking is also reduced, since otherwise this easily occurs with the connection of the various components.

A simple construction with a view to manufacturing is achieved if the Y-piece is formed from two bowl-shaped parts and the bacteria filter is clamped between these parts substanially perpendicular to the through-flow direction.

The Y-piece of the connection arrangement normally includes three attachment nipples, namely one for the patient attachment piece, one for the inhalation tube and one for the exhalation tube. From a manufacturing point of view a particularly suitable construction is obtained when these nipples are arranged substanially parallel to each other.

Preferably the Y-piece's through-flow area is maximized in proportion to its volume through a substanially circular form, whilst its length in the flow direction is restricted to that which the function allows.

The sample withdrawal outlet is suitably arranged in the form of a nipple substanially in the middle of a Y-piece with, in a direction perpendicular to the through-flow direction, a somewhat drawn out through-flow area, for example substanially oval or rounded-rectangular shaped, between the attachment nipples for the inhalation resp exhalation tubes and preferably parallel to these nipples. Alternatively the sample withdrawal outlet can have the form of a nipple which is angled in respect to the other nipples. Important in both cases is, however, that it is shieldably arranged between the attachment nipples for the inhalation resp exhalation tubes.

The Y-piece requires a certain minimum volume. By way of example the included attachment nipples should have a certain standard dimension. In order that this volume affects the withdrawal sample as little as possible, the sample withdrawal outlet is connected to the first sample withdrawal tube which extends from the outlet to the bacteria filter. This sample withdrawal tube can, as mentioned above, via the bacteria filter, be connected to a second sample withdrawal tube which extends from the filter towards the patient attachment piece and preferably up to and, possibly, into this piece. In this way the effect of the Y-piece's inner volume on the withdrawal sample is substantially eliminated.

The sample withdrawal is further simplified when said first and second sample withdrawal tubes are arranged on either side of the bacteria filter and are in pressured contact therewith via an at right angles to the flow direction widened portion, for example a cone-shaped and/or cylindrical funnel-like portion. In this way the simplification of, amongst other things, the flow of the sample through the bacteria filter is achieved, whilst leakage is prevented between the inner sample withdrawal tube and the atmosphere surrounding it.

To secure the position of, and the contact between, said sample withdrawal tubes, one or both bowl-shaped halves of the Y-piece can be provided with support webs for one or both tubes.

Should the connection arrangement serve at the same time as a heat and moisture exchanger by being equipped with a wad, or similar, of the above-mentioned type, then this should suitably be impregnated with an antibacterial agent, for example chlorhexidine or hydrogen peroxide, and/or with a hygroscopic substance, such as magnesium chloride, lithium chloride or calcium chloride.

In a preferred embodiment of the connection arrangement according to the invention, the above-mentioned wad or similar consists of fibres of a plastic material having a certain melting point, such as polypropylene, which are coated with another plastic material having a lower melting point, such as polyethylene, with the aid of which the fibres are bounded by heating to said lower melting point. In this way the breaking off of material from the fibre wad and its transmittal in to the patient's respiratory organs is effectively prevented.

Preferably at least one part of the patient attachment piece is made from transparent material. This part, which preferably is located nearest the patient, is left free from other material in order to serve as a secretion trap and/or inspection zone.

In certain circumstances, for example in connection with anaesthesia treatment, the patient attachment piece is not required. This is therefore suitably arranged to be disconnectable from the Y-piece.

Said sample withdrawal outlet is appropriately arranged in a dome, or similar, directed towards the interior of the Y-piece and which is arranged to stabilize the outlet whilst also reducing the inner volume of the Y-piece.

Preferably both bowl-shaped parts of the Y-piece are provided with internal stiffening webs which are so arranged that they disturb the through-flow as little as possible, for example by being radially directed in relation to the principal through-flow direction, whilst also being arranged to support the bacteria filter on both sides.

Due to the fact that the present invention, according to the applicant, is closely related to the invention according to co-pending European patent application 91.108316.0 (EP 462 412) (parent application to the present divisional application) both related to the same product, the product in its entirety is described in the following, inclusive modifications. In such a way both inventions can be easier understood.

### DESCRIPTION OF THE DRAWINGS

Figure 1 shows a section through a Y-piece according to the invention.

Figure 2 shows the same Y-piece as in Figure 1, but from above.

Figure 3 shows a somewhat modified Y-piece.

Figure 4 shows the last-mentioned Y-piece seen from underneath.

Figure 5 shows schematically a somewhat more complete connection arrangement according to the invention.

Figure 6 shows a plan view of the connection arrangement according to Figure 5.

Figure 7 shows a section through a nipple and a side view of another nipple belonging to the Y-piece which is included in the connection arrangement according to Figure 5.

Figure 8 shows a section along line VIII-VIII in Figure 5, but without the filter.

Figures 9-12 show views corresponding to Figures 5-8 of a modified embodiment of the connection arrangement according to the invention.

Figure 13 shows a further embodiment of a Y-piece. At the same time fig 13a shows, on a larger scale, a part of fig 13.

Figure 14 shows the Y-piece according to Figure 13 seen from beneath and Figure 15 shows the same seen from above.

### BEST MODE OF CARRYING OUT THE INVENTION

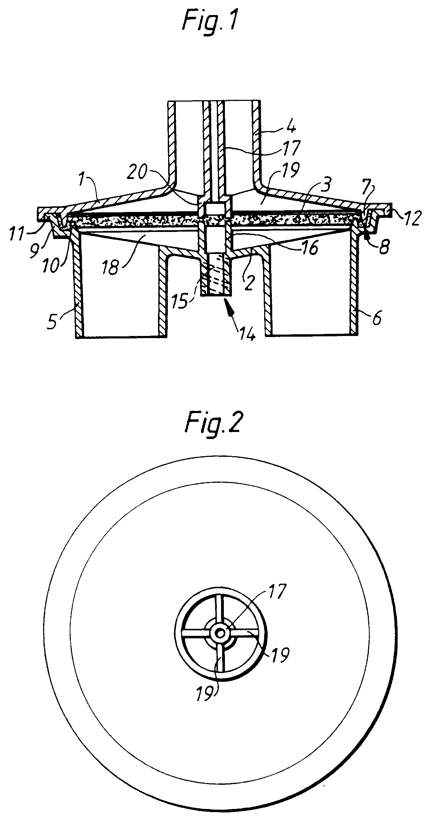

Figures 1 and 2 show a section resp end view of a Y-piece according to the invention which is intended to be included in a connection arrangement also according to the invention.

This Y-piece comprises two bowl-shaped parts 1 and 2 with a bacteria filter 3 clamped therebetween. The upper bowl-shaped part 1 is provided with a nipple 4 which is intended to be connected to a patient attachment piece. The lower bowl-shaped part 2 is provided with two parallel nipples 5 and 6 which are intended to be connected to an inhalation tube resp an exhalation tube, which in turn are intended to be connected to a respirator, anaesthesia machine or similar.

The bacteria filter 3 is clamped between a circular ridge 7 on the upper bowl-shaped part 3 and a circular groove 8 in the lower bowl-shaped part 2. This groove 8 is formed from an outer flange 9 and an inner ridge 10. The flange 9 is terminated at its outer portion by a radially extending partial flange 11 which is intended to be fixed to the upper bowl-shaped part 1 by, for example, glueing or welding , preferably ultrasonic welding. The concentricity of the two bowl-shaped parts 1 and 2 is hereby facilitated by an outer peripheral flange 12 on the upper bowl-shaped part 1.

The lower bowl-shaped part 2 further comprises a sample withdrawal outlet in the form of a nipple 14, which is preferably provided with an inner or outer screw thread or other fixing means 15. The nipple 14 is connected to a first sample withdrawal tube 16 which in turn, via filter 3, is connected to a second sample withdrawal tube 17. Both the sample withdrawal tubes and the filter 3 are supported by radially extending support webs 18 resp 19. The sample withdrawal tube 17 is terminated inside with a widened cylindrical portion 20 which via the filter presses against a corresponding portion of the sample withdrawal tube 16. As is evident from Figure 2, the sample withdrawal tube 17 is located between four radially directed support webs 19.

### ALTERNATIVE EMBODIMENTS OF THE INVENTION

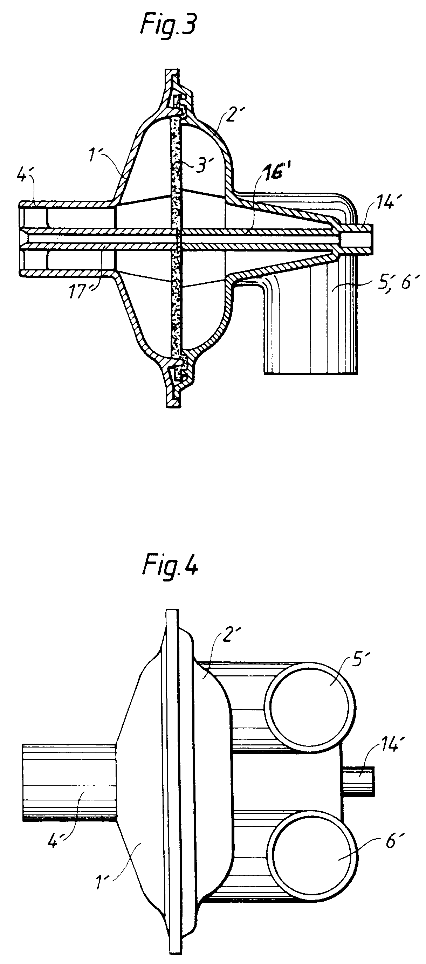

In Figures 3 and 4 there is shown a modified embodiment of a Y-piece in the connection arrangement according to the invention. The construction corresponds in principle with that according to Figure 1 and 2. Thus the same reference numerals have been used but with the addition of a dash. The most significant difference is that the attachment nipples 5' resp 6' are angularly formed. Furthermore the sample withdrawal tubes 16' and 17' have been given a somewhat different shape.

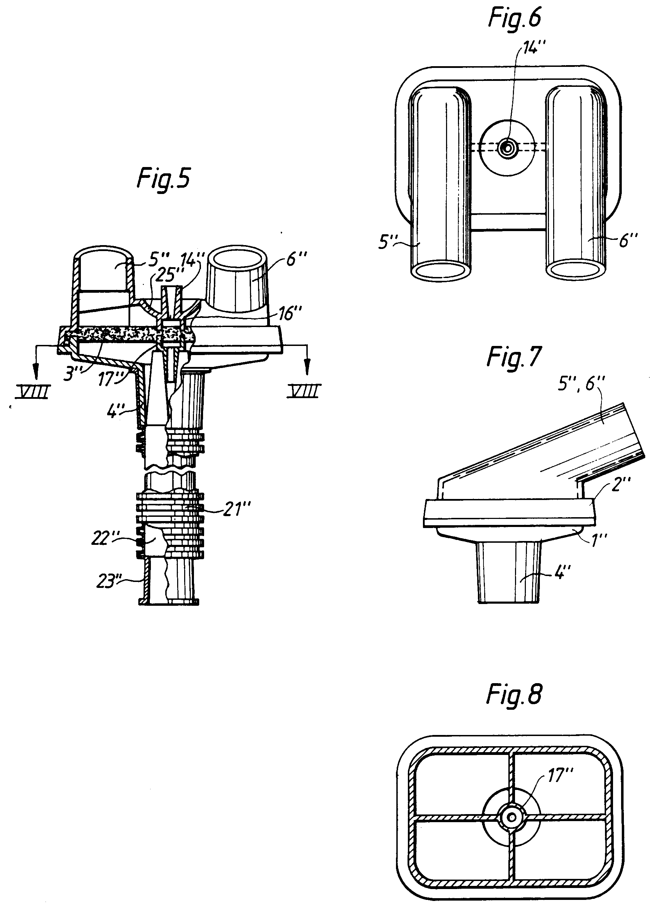

In Figures 5-8 there is shown a somewhat more complete realization of an embodiment of the connection arrangement according to the invention. This also principally corresponds with that which has been described above. Thus the same figure reference numerals are used for corresponding details, but with the addition of a double dash. Reference numerals 5'' resp 6'' thus denotes two nipples which are intended to be attached to an inhalation tube resp an exhalation tube. The bacteria filter itself is denoted by 3''. Reference numeral 14'' denotes a sample withdrawal nipple which is connected to a first sample withdrawal tube 16''. The latter is in turn via the filter 3'' in contact with a second sample withdrawal tube 17''. Reference numeral 4'' denotes a nipple with the aid of which the Y-piece is connected to a patient attachment piece 21''. This is preferably designed substanially in accordance with that described in US-A-4 516 573. It is, however, preferably of uniform thickness. Within the patient attachment piece there is preferably a wad or similar 22'' of a moisture and heat absorbing material which serves to take up heat and moisture from the exhaled gas and pass this to the inhaled gas. Finally in Figure 5, reference numeral 23'' denotes an attachment pipe or cone with the help of which the patient attachment piece 21'' can be connected to a tracheal tube or similar.

The Y-piece shown in Figures 5-8 also consists of two bowl-shaped parts 1'' resp 2'' but differs from the above described Y-pieces in that, for example these parts have been given a rounded rectangular shape. Furthermore the attachment nipples 5'' and 6'' are arranged at a different angle to that which is shown in, for example, Figure 3 and 4. Finally, reference numerals 25'' denotes a dome directed towards the interior of the Y-piece in which the sample withdrawal nipple 14'' is arranged. Thanks to this arrangement the Y-piece's inner volume is reduced whilst the sample withdrawal tube 16'' is stabilized.

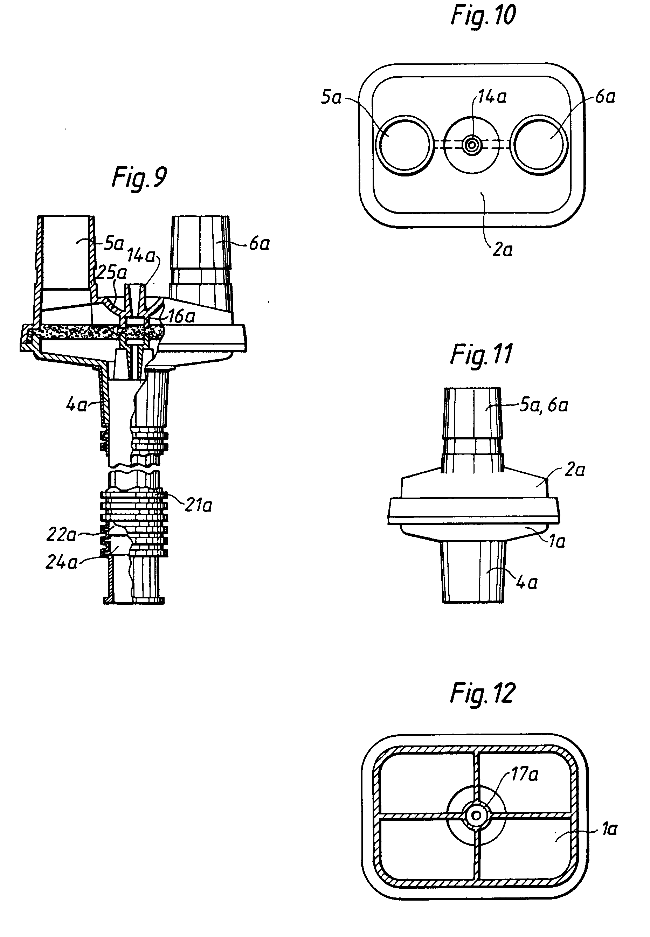

The construction according to Figures 9-12 substantially corresponds with that according to Figures 5-8. Corresponding details have thus been given the same figure reference numerals, but with the suffix a instead of the double dashes used in Figures 5-8. The construction according to Figures 9-12 differs from that according to Figures 5-8 in that the nipples 5a and 6a are arranged parallel to the nipple 4a. Furthermore the fibre wad 22a does not fill the whole patient attachment piece 21a. A free space 24a is left nearest the patient, which is intended to serve as a secretion trap and which can also serve as an inspection zone if the patient attachment piece is made from a transparent material. Finally, reference numeral 25a denotes a dome directed towards the interior of the Y-piece in which the sample withdrawal nipple 14a is arranged. Thanks to this arrangement the Y-piece's inner volume is reduced whilst the sample withdrawal tube 16a is stabilized.

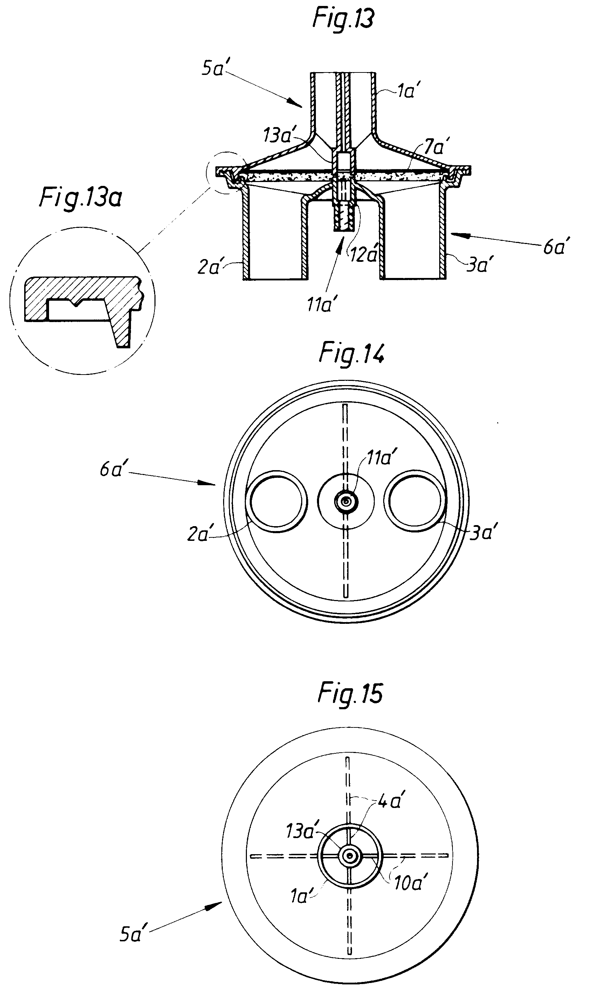

Figures 13-15 are showing a further alternative for the Y-piece made in accordance with the invention. This embodiment corresponds essentially to the one shown in Figures 1 and 2. Different reference numerals have, however, been used, but all with the addition of a'. The Y-piece described comprises two bowl-shaped parts 5a' and 6a' with a bacteria filter 7a' clamped therebetween. The upper bowl-shaped part 5a' is provided with a nipple 1a', which is intended to be connected to a patient attachment piece. The lower bowl-shaped part 6a' is provided with two parallel nipples 2a' and 3a' which are intended to be connected to an inhalation tube resp exhalation tube, which in turn are intended to be connected to a respirator, anaesthesia machine or similar.

The bacteria filter 7a' is clamped in the same way as the filter 3 in fig 1. The lower bowl-shaped part 6a' further comprises a sample withdrawal outlet in the form of a nipple 11a'. The nipple 11a' is connected to a first sample withdrawal tube 12a' which in turn, via the filter 7a', is connected to a second sample withdrawal tube 13a'.

The main difference between the embodiment according to Figures 1-2 and the one according to Figures 13-15 is that the last mentioned embodiment is provided with a partition wall 4a', preferably made in one piece with the two sample withdrawal tubes 12a' and 13a', separating exhaled gas from inhaled gas. As shown in Figure 15 the part 5a' may also be provided with a support wall 10a' intended to support the filter 7a'.

## Claims

1. Y-piece, for use in a connection arrangement for connecting a patient to a respirator, anaesthesia machine or similar, wherein said Y-piece comprises a sample withdrawal outlet (14) **characterized** in that a bacteria filter (3) is mounted in said Y-piece substantially perpendicular to the through-flow direction, and that the sample withdrawal outlet (14) is arranged in the exhalation flow direction after the bacteria filter (3) and is connected to a first sample withdrawal tube (16) which extends from the bacteria filter (3) to the outlet (14) and is arranged so as to take bacteria-free samples through said filter (3).

2. Y-piece according to claim 1, **characterized** in that said first sample withdrawal tube (16) via the bacteria filter (3) is connected to a second sample withdrawal tube (17) which extends from the bacteria filter (3) towards a nozzle (4, 4', 4'', 4a) connectable to a patient attachment piece (21''; 21a) and preferably extends through this nozzle (4) up to and, possibly, into this piece.

3. Y-piece according to claim 2, **characterized** in that said first and second sample withdrawal tubes (16,17) are arranged on either side of the bacteria filter (3) and are in pressurized contact therewith via an at right angles to the flow direction widened portion, for example a cone-shaped and/or cylindrical funnel-like portion (20).

4. Y-piece according to any of the preceding claims, **characterized** in that the Y-piece is provided with a partition wall (4a') separating the exhaled gas from the inhaled gas, and extending in the direction towards the patient up to at least the bacteria filter (7a').

5. Y-piece according to claims 2-4, **characterized** in that the through-flow area of the Y-piece (1,2) is enlarged in comparison with that of the connectable patient attachment piece.

6. Y-piece according to any of the preceding claims wherein the Y-piece (1,2) comprises three attachment nipples, namely one (4) for the patient attachment piece, one (5) for the inhalation tube and one (6) for the exhalation tube, **characterized** in that said nipples (4-6) are arranged substantially parallel to each other.

7. Y-piece according to any of the preceding claims, **characterized** in that the Y-piece's (1,2) through-flow area is maximized in proportion to its volume through a substantially circular form, whilst its length in the flow direction is restricted to that which the function allows.

8. Y-piece according to claim 6, **characterized** in that the sample withdrawal outlet (14'' or 14a) is arranged, preferably in a form of a nipple, substanially in the middle of the Y-piece (1'', 2''; 1a, 2a) between the attachment nipples (5'', 6''; 5a, 6a) for the inhalation resp exhalation tubes and preferably parallel to these nipples, whereby the sample withdrawal outlet (14'', 14a)has, in a direction perpendicular to its through-flow direction, a somewhat drawn-out through-flow area, for example a substantially oval or rounded-rectangular shaped through-flow area.

9. Y-piece according to any of the preceding claims, **characterized** in that the Y-piece comprises two bowl-shaped halves(1,2), wherein one or both of said bowl-shaped halves (1,2) are provided with support webs (18,19) for said first and/or second sample withdrawal tube.

10. Y-piece according to claim 9, **characterized** in that the sample withdrawal outlet (14'', 14a) is arranged in a dome, or similar, directed towards the interior of the Y-piece (1,2) and which is arranged to stabilize the outlet whilst also reducing the inner volume of the Y-piece.

11. Y-piece according to any one of the claims 9-10, **characterized** in that both bowl-shaped parts of the Y-piece (1,2) are provided with support webs (18, 19) which are so arranged that they disturb the through-flow as little as possible, for example by being radially directed in relation to the principal through-flow direction, whilst also being arranged to support the bacteria filter (3) on both sides.

12. Connection arrangement for connecting a patient to a respirator, anaesthesia machine or similar, comprising a patient attachment piece (21'' or 21a) and a so-called Y-piece (1,2), arranged to join the patient attachment piece to an inhalation resp exhalation tube **characterized** in that said Y-piece is a Y-piece according to any of the previous claims.

13. Connection arrangement according to claim 12, **characterized** in that the patient attachment piece (21'' or 21a) is flexible and contains a heat and moisture exchange system in the form of a wad or similar (22'' or 22a) of a flexible material, such as fibers, with the ability to take up heat and moisture from exhaled gas and subsequently deliver this to the inhaled gas, wherein said wad, or similar, (22'', 22a) is impregnated with an antibacterial agent, for example chlorhexidine or hydrogen peroxide, and/or with a hygroscopic substance, such as magnesium chloride, lithium chloride or calcium chloride.

14. Connection arrangement according to claim 13, **characterized** in that said wad or similar (22'', 22a) consists of fibres of a plastic material having a certain melting point, such as polypropylene, which are coated with another plastic material having a lower melting point, such as polyethylene, with the aid of which the fibres are bonded by heating to said lower melting point.

15. Connection arrangement according to any of the claims 12-14, **characterized** in that at least one part (24a) of the patient attachment piece (21a) is transparent and no other material is in this part, preferably nearest the patient, so that it can serve as a secretion trap and/or inspection zone.

16. Connection arrangement according to any of the claims 12-16, **characterized** in that the patient attachment piece (for example 21'' or 21a) is disconnectable from the Y-piece (1,2).

## Patentansprüche

1. Y-Stück zur Verwendung in einer Verbindungsanordnung zum Verbinden eines Patienten mit einem Beatmungsgerät, einer Narkosemaschine oder ähnlichem, bei dem das Y-Stück einen Probenabführungsauslaß (14) aufweist, dadurch gekennzeichnet, daß ein Bakterienfilter (3) in dem Y-Stück im wesentlichen rechtwinklig zu der Durchströmungsrichtung angebracht ist, und daß der Probenabführungsauslaß (14) in der Ausatmungsströmungsrichtung hinter dem Bakterienfilter (3) angeordnet und mit einer ersten Probenabführungsröhre (16) verbunden ist, die sich von dem Bakterienfilter (3) zu dem Auslaß (14) erstreckt und derart angeordnet ist, daß sie bakterienfreie Proben über das Filter (3) aufnimmt.

2. Y-Stück nach Anspruch 1, dadurch gekennzeichnet, daß die erste Probenabführungsröhre (16) über das Bakterienfilter (3) mit einer zweiten Probenabführungsröhre (17) verbunden ist, die sich von dem Bakterienfilter (3) bis zu einer Düse (4, 4', 4'', 4a), die mit einem Patientenverbindungsteil (21''; 21 a) verbindbar ist und die sich vorzugsweise durch diese Düse (4) hindurch und möglicherweise bis in dieses Teil erstreckt.

3. Y-Stück nach Anspruch 2, dadurch gekennzeichnet, daß die erste und die zweite Probenabführungsröhre (16, 17) jeweils auf einer Seite des Bakterienfilters (3) angeordnet sind und mit diesem über einen rechtwinklig zu der Strömungsrichtung verbreiterten Abschnitt, beispielsweise einen kegelförmigen und/oder einen trichterförmigen Abschnitt (20), in Druckberührung stehen.

4. Y-Stück nach einem der vorhergehenden Ansprüche, dadurch gekennzeichnet, daß das Y-Stück mit einer Trennwand (4a') versehen ist, die das ausgeatmete Gas von dem eingeatmeten Gas trennt und sich in der Richtung zu dem Patienten bis mindestens zu dem Bakterienfilter (7a') erstreckt.

5. Y-Stück nach den Ansprüchen 2 bis 4, dadurch gekennzeichnet, daß die Durchströmungsfläche des Y-Stücks (1, 2) im Vergleich mit derjenigen des verbindbaren Patientenverbindungsteils vergrößert ist.

6. Y-Stück nach einem der vorhergehenden Ansprüche, bei dem das Y-Stück (1, 2) drei Anbringungsnippel aufweist, nämlich einen (4) für das Patientenverbindungsteil, einen (5) für den Inhalationsschlauch und einen (6) für den Ausatmungsschlauch, dadurch gekennzeichnet, daß die Nippel (4 bis 6) im wesentlichen parallel zueinander angeordnet sind.

7. Y-Stück nach einem der vorhergehenden Ansprüche, dadurch gekennzeichnet, daß die Durchströmungsfläche des Y-Stücks (1, 2) in Relation zu seinem Volumen aufgrund einer im wesentlichen kreisförmigen Form maximiert ist, wohingegen seine Länge in der Strömungsrichtung auf diejenige Länge, die die Funktion ermöglicht, beschränkt ist.

8. Y-Stück nach Anspruch 6, dadurch gekennzeichnet, daß der Probenabführungsauslaß (14'' oder 14a) vorzugsweise in der Form eines Nippels im wesentlichen in der Mitte des Y-Stücks (1'', 2''; 1a, 2a) zwischen den Anbringungsnippeln (5'', 6''; 5a, 6a) für den Inhalationsschlauch bzw. den Ausatmungsschlauch sowie vorzugsweise parallel zu diesen Nippeln angeordnet ist, so daß der Probenabführungsauslaß (14'', 14a) in der rechtwinklig zu seiner Durchströmungsrichtung verlaufenden Richtung eine etwas herausgezogene Durchströmungsfläche, beispielsweise eine im wesentlichen ovale oder mit abgerundeten Ecken versehene rechteckförmige Durchströmungsfläche aufweist.

9. Y-Stück nach einem der vorhergehenden Ansprüche, dadurch gekennzeichnet, daß das Y-Stück zwei schalenförmige Hälften (1, 2) aufweist, wobei eine oder beide dieser schalenförmigen Hälften (1, 2) mit Stützstegen (18, 19) für die erste und/oder die zweite Probenabführungsröhre versehen sind.

10. Y-Stück nach Anspruch 9, dadurch gekennzeichnet, daß der Probenabfühtungsauslaß (14'', 14a) in einem Dom oder ähnlichem angeordnet ist, der in Richtung zu dem Inneren des Y-Stücks (1, 2) gerichtet ist, und der derart angeordnet ist, daß er den Auslaß stabilisiert, wobei er zugleich auch das Innenvolumen des Y-Stücks verringert.

11. Y-Stück nach einem der Ansprüche 9 oder 10, dadurch gekennzeichnet, daß die beiden schalenförmigen Teile des Y-Stücks (1, 2) mit Stützstegen (18, 19) versehen sind, die derart angeordnet sind, daß sie die Durchströmung so wenig wie möglich stören. indem sie beispielsweise mit Bezug zu der hauptsächlichen Strömungsrichtung radial ausgerichtet sind, wobei sie ferner derart angeordnet sind, daß sie das Bakterienfilter (3) an dessen beiden Seiten halten.

12. Verbindungsanordnung zur Verbindung eines Patienten mit einem Beatmungsgerät, einer Narkosemaschine oder ähnlichem, mit einem Patientenverbindungsteil (21'' oder 21a) und einem sogenannten Y-Stück (1, 2), das derart angeordnet ist, daß es das Patientenverbindungsteil mit einem Inhalationsschlauch bzw. Ausatmungsschlauch verbindet, dadurch gekennzeichnet, daß das Y-Stück ein Y-Stück gemäß einem der vorhergehenden Ansprüche ist.

13. Verbindungsanordnung nach Anspruch 12, dadurch gekennzeichnet, daß das Patientenverbindungsteil (21'' oder 21a) flexibel ist und ein Wärme- und Dampfaustauschsystem in der Form eines Bündels oder ähnlichem (22'' oder 22a) aus einem flexiblen Material, wie etwa aus Fasern, enthält, das die Fähigkeit besitzt, Wärme und Feuchtigkeit aus dem ausgeatmeten Gas aufzunehmen und diese nachfolgend zu dem inhalierten Gas zu leiten, wobei das Bündel oder dergleichen (22'', 22a) mit einem antibakteriellen Mittel, z. B. Chlorhexidin oder Wasserstoffperoxid, und/oder mit einer hygroskopischen Substanz, wie etwa Magnesiumchlorid, Lithiumchlorid oder Kalziumchlorid imprägniert ist.

14. Verbindungsanordnung nach Anspruch 13, dadurch gekennzeichnet, daß das Bündel oder ähnliches (22'', 22a) aus Fasern aus einem Kunststoffmaterial mit einem gewissen Schmelzpunkt, wie etwa aus Polypropylen besteht, die mit einem weiteren Kunststoffmaterial beschichtet sind, das einen niedrigeren Schmelzpunkt aufweist, wie etwa mit Polyethylen, mit dessen Hilfe die Fasern dadurch miteinander verbunden werden, daß sie auf den niedrigeren Schmelzpunkt erwärmt werden.

15. Verbindungsanordnung nach einem der Ansprüche 12 bis 14, dadurch gekennzeichnet, daß mindestens ein Teil (24a) des Patientenverbindungsteils (21a) transparent ist und sich in diesem Teil kein weiteres Material, vorzugsweise so nahe wie möglich bei dem Patienten, befindet, so daß es als Sekretfalle und/oder als Inspektionszone dienen kann.

16. Verbindungsanordnung nach einem der Ansprüche 12 bis 15, dadurch gekennzeichnet, daß das Patientenverbindungsteil (z. B. 21'' oder 21a) von dem Y-Stück (1,2) abtrennbar ist.

## Revendications

1. Pièce en Y destinée à être utilisée dans un dispositif de connexion servant à raccorder un patient à un respirateur, une machine d'anesthésie ou similaire, ladite pièce en Y comprenant une sortie (14) d'extraction d'échantillons, caractérisée en ce qu'un filtre (3) à bactéries est monté dans ladite pièce en Y de façon sensiblement perpendiculaire à la direction d'écoulement traversant, et en ce que la sortie (14) d'extraction d'échantillons est disposée, dans le sens d'écoulement d'exhalaison, après le filtre (3) à bactéries et est connectée à un premier tube (16) d'extraction d'échantillons, qui s'étend depuis le filtre (3) à bactéries jusqu'à la sortie (14) et est agencé de manière à prélever des échantillons exempts de bactéries à travers ledit filtre (3);

2. Pièce en Y selon la revendication 1, caractérisée en ce que ledit premier tube (16) d'extraction d'échantillon est raccordé, par l'intermédiaire du filtre (3) à bactéries, à un deuxième tube (17) d'extraction d'échantillons qui s'étend à partir du filtre (3) à bactéries en direction d'un ajutage (4, 4', 4'', 4a) pouvant être raccordé à une pièce (21''; 21a) de fixation à un patient et qui s'étend de préférence à travers cet ajutage (4) jusqu'à cette pièce et, éventuellement, jusque dans cette pièce.

3. Pièce en Y selon la revendication 2, caractérisée en ce que lesdits premier et deuxième tubes (16, 17) d'extraction d'échantillons sont disposés de part et d'autre du filtre (3) à bactéries et sont en contact sous pression avec ce dernier, par l'intermédiaire d'une partie élargie perpendiculairement à la direction d'écoulement, par exemple une partie (20) analogue à un entonnoir conique et/ou cylindrique.

4. Pièce en Y selon l'une quelconque des revendications précédentes, caractérisée en ce que la pièce en Y est pourvue d'une paroi formant cloison (4a') séparant le gaz exhalé du gaz inhalé, et s'étendant en direction du patient jusqu'au moins le filtre (7a') à bactéries.

5. Pièce en Y selon les revendications 2-4, caractérisée en ce que la zone d'écoulement traversant de la pièce en Y (1, 2) est agrandie en comparaison de celle de la pièce de fixation au patient.

6. Pièce en Y selon l'une quelconque des revendications précédentes, cette pièce en Y (1, 2) comprenant trois embouts de fixation, à savoir un embout (4) pour la pièce de fixation au patient, un embout (5) pour le tube d'inhalation et un embout (6) pour le tube d'exhalaison, caractérisée en ce que lesdits embouts (4-6) sont disposés de façon sensiblement parallèle les uns par rapport aux autres.

7. Pièce en Y selon l'une quelconque des revendications précédentes, caractérisée en ce que la zone d'écoulement traversant de la pièce en Y (1, 2) est maximisée en proportion de son volume par une forme sensiblement circulaire, tandis que sa longueur dans le direction d'écoulement est limitée à celle que permet le fonctionnement.

8. Pièce Y selon la revendication 6, caractérisée en ce que la sortie (14'' ou 14a) d'extraction d'échantillons est conçue, de préférence, sous la forme d'un embout, sensiblement au milieu de la pièce Y (1'', 2''; 1a, 2a) entre les embouts de fixation (5'', 6''; 5a, 6a) destinés respectivement aux tubes d'inhalation et d'exhalaison et de préférence parallèlement à ces embouts, grâce à quoi la sortie (14'', 14a) comporte dans une direction perpendiculaire à sa direction d'écoulement traversant, une zone d'écoulement traversant quelque peu étirée, par exemple une zone d'écoulement traversant de forme ovale ou rectangulaire arrondie.

9. Pièce en Y selon l'une quelconque des revendications précédentes, caractérisée en ce qu'elle comprend deux moitiés (1, 2) en forme de bols, l'une ou les deux moitiés précitées (1, 2) en forme de bols étant munies d'âmes de support (18, 19) pour lesdits premier et deuxième tubes d'extraction d'échantillons.

10. Pièce en Y selon la revendication 9, caractérisée en ce que la sortie (14'', 14a) d'extraction d'échantillons est disposée dans un dôme, ou similaire, dirigé vers l'intérieur de la pièce en Y (1, 2) et qui est conçu de manière à stabiliser la sortie tout en réduisant aussi le volume intérieur de la pièce en Y.

11. Pièce en Y selon l'une quelconque des revendications 9-10, caractérisée en ce que les deux parties en forme de bols de la pièce en Y (1, 2) sont pourvues d'âmes de support (18, 19) qui sont disposées de manière telle qu'elles perturbent le moins possible l'écoulement traversant, par exemple par le fait qu'elles sont dirigées radialement par rapport à la direction principale d'écoulement traversant tout en étant agencées de manière à supporter le filtre (3) à bactéries sur les deux côtés.

12. Dispositif de connexion destiné à connecter un patient à un respirateur, une machine d'anesthésie ou similaire, comprenant une pièce (21'' ou 21a) de fixation à un patient et ce que l'on appareil un pièce en Y (1, 2) conçue pour raccorder la pièce de fixation à un tube d'inhalation et un tube d'exhalaison ,respectivement, caractérisé en ce que ladite pièce en Y est une pièce en Y selon l'une quelconque des revendications précédentes.

13. Dispositif de connexion selon la revendication 12, caractérisée en ce que la pièce (21'' ou 21a) de fixation à un patient est flexible et contient un système d'échange de chaleur et d'humidité sous la forme d'un tampon ou similaire (22'' ou 22a) d'une matière flexible, telle que des fibres, capable d'extraire la chaleur et l'humidité du gaz exhalé et de délivrer ensuite cette chaleur et cette humidité au gaz inhalé, ledit tampon, ou similaire, (22'', 22a) étant imprégné d'un agent antibactérien, par exemple de la chlorhexidine ou du peroxyde d'hydrogène, et/ou d'une substance hygroscopique, telle que du chlorure de magnésium, du chlorure de lithium ou du chlorure de calcium.

14. Dispositif de connexion selon la revendication 13, caractérisée en ce que ledit tampon, ou similaire, (22'', 22a) consiste en des fibres de matière plastique qui ont un certain point de fusion, tel que du polypropylène, et qui sont revêtues avec une autre matière plastique ayant un point de fusion inférieur, telle que du polyéthylène à l'aide de laquelle on lie les fibres en les chauffant jusqu'audit point de fusion inférieur.

15. Dispositif de connexion selon l'une quelconque des revendications 12-14, caractérisé en ce qu'au moins une partie (24a) de la pièce (21a) de fixation à un patient est transparente et qu'aucune autre matière ne se trouve dans cette partie, de préférence à un endroit situé le plus près du patient, de sorte qu'elle peut servir de piège à secrétion et/ou de zone d'inspection.

16. Dispositif de connexion selon l'une quelconque des revendications 12-16, caractérisé en ce que la pièce de fixation à un patient (par exemple 21'' ou 21a) peut être déconnectée de la pièce Y (1,2).
